# EUROPÄISCHE PATENTSCHRIFT

(11) **EP 2 289 873 B1**
(45) Veröffentlichungstag und Bekanntmachung des Hinweises auf die Patenterteilung: **08.02.2012**
(21) Anmeldenummer: 09169055.2
(22) Anmeldetag: 31.08.2009
(51) Int. Cl.: C07C 269/06, C07C 271/12

(54) **Verfahren zur Herstellung von Iodpropargylverbindungen**
Process for the preparation of iodopropargyl compounds
Procédé de préparation de composés iodopropargyles

(43) Veröffentlichungstag der Anmeldung: 02.03.2011
(73) Patentinhaber: LANXESS Deutschland GmbH, 51369 Leverkusen (DE)
(72) Erfinder: Koop, Bernd, Dr., 50668 Köln (DE); Gausing, Wolfgang, Dr., 40885 Ratingen (DE); Uhr, Hermann, Dr., 51373 Leverkusen (DE)

(56) Entgegenhaltungen:
- EP-A- 0 014 032
- EP-A- 0 513 541
- US-A- 4 330 521
- R.O. NORRIS ET AL.: JOURNAL OF THE AMERICAN CHEMICAL SOCIETY, vol. 61, 1939, pages 1460-1461,
- G.F. HENNION ET AL.: JOURNAL OF THE AMERICAN CHEMICAL SOCIETY, 1940, pages 1368-1371,

## Beschreibung

Die vorliegende Erfindung betrifft ein neues Verfahren zur Herstellung von Iodpropargylverbindungen.

Iodpropargylverbindungen sind bekannte Wirkstoffe, die besonders im Materialschutz dazu eingesetzt werden, technische Materialien wie Klebstoffe, Leime, Papier und Karton, Textilien, Leder, Holz, Holzwerkstoffe, Anstrichmittel und Kunststoffartikel, Kühlschmierstoffe und andere Materialien, die von Mikroorganismen befallen oder zersetzt werden können vor dem Befall, besonders durch Pilze zu schützen. Bekanntester Vertreter ist IPBC (3-Iod-2-propinyl-N-butylcarbamat).

Von besonderer Bedeutung ist es, bei der Herstellung sehr sauberes IPBC zu erhalten, da mehrfach beispielsweise zwei- oder dreifach iodierte Verbindungen, die sehr leicht bei der Iodierung entstehen können, zu einer unerwünschten Verfärbung führen, die sich bei der Verwendung als Mikrobizid negativ bemerkbar machen. Des Weiteren führen diese Verunreinigungen häufig dazu, dass sich mit IPBC geschützte Materialien durch Lichteinwirkung verfärben.

Es sind bereits einige Verfahren zur Herstellung von Iodpropargylverbindungen, insbesondere von IPBC, bekannt.

DE-A-2433410 beschreibt beispielsweise die Iodierung von Propargylalkohol mit Iod, wobei Natriumhypochlorit als Oxidationsmittel für das entstehende Iodid verwendet wird. Daran schließt sich die Umsetzung des iodierten Propargylalkohols mit Alkylisocyanaten zu Iodpropargylverbindungen an.

EP-A-14032 beschreibt die Umsetzung von Propargylalkohol mit Alkylisocyanaten zu Propargylalkylcarbamaten und anschließender Iodierung mit Iod oder Metalliodiden unter Verwendung von NaOCl als Oxidationsmittel zu Iodpropargylverbindungen. Als Lösungsmittel ist die Verwendung von Wasser unter Zusatz eines Cosolvens (z.B. Methanol) oder eines Tensids bzw. Lösungsvermittlers (z.B. partiell hydrolysiertes Polyvinylacetat) beschrieben.

Die Iodierung von Propargylverbindungen ist in US-A-5693849 in wässrigem Medium mit Iod und Natriumhypochlorit unter Zusatz eines grenzflächenaktiven Stoffes beschrieben, wobei es sich dabei um einen acidischen Teilester eines organischen Phosphats oder das Salz eines sulfatierten Fettalkohols handelt.

W02005/016871 beschreibt die Iodierung von Propargylbutylcarbamat (PBC) im wässrigen Medium unter Zusatz eines nichtionischen Tensids mit Metalliodiden und Natriumhypochlorit als Oxidationsmittel zu IPBC.

Bei allen bekannten Verfahren wird Natriumhypochlorit als Oxidationsmittel eingesetzt. Dies ist notwendig, um entstehendes oder eingesetztes Iodid zu oxidieren und damit die Iodquelle effektiv nutzen zu können. Da die verwendete Natriumhypochlorit-Lösung üblicherweise eine Konzentration von lediglich 5-15% hat, führt dies zu einer ungewollten Verdünnung der Reaktionslösung und dadurch nach Isolierung des Produktes zu einer größeren Menge an Abwasser, das zu hohen Kosten entsorgt werden muss. Des Weiteren hat Natriumhypochlorit den Nachteil, dass es sich leicht zersetzt, wodurch die Konzentration abnimmt. Damit nimmt die ungewollte Verdünnung weiter zu.

Daher müssen Natriumhypochlorit-Lösungen sehr sorgfältig gelagert werden. Darüber hinaus können bei der Herstellung von Natriumhypochlorit Nebenprodukte entstehen, z.B. Natriumchlorat, die ebenfalls bei der Verwendung von Natriumhypochlorit-Lösungen zu unerwünschten Nebenreaktionen führen können.

Aufgabe war es daher ein Verfahren zu finden, bei dem diese Nachteile nicht auftreten.

Überraschenderweise wurde ein Verfahren zur Herstellung von Iodpropargylverbindungen der Formel (I) gefunden, 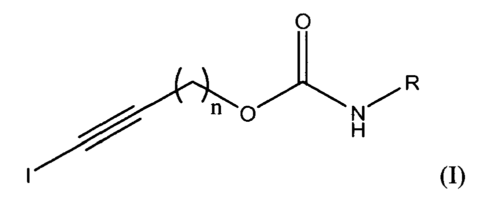 worin R für Wasserstoff, jeweils gegebenenfalls substituiertes C₁-C₂₀-Alkyl, C₂-C₂₀-Alkenyl, C₆-C₂₀-Aryl oder C₃-C₂₀-Cycloalkyl
und n für eine ganze Zahl von 1 bis 6 steht,
dadurch gekennzeichnet, dass man Propargylverbindungen der Formel (II) 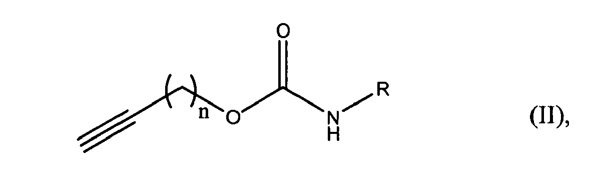 worin R und n die obige Bedeutung haben, mit Iod und/oder Metalliodiden in Gegenwart einer Base und unter Verwendung von Chlor umsetzt.

Bevorzugte gegebenenfalls substituierte C₂-C₂₀-Alkenyle in der Bedeutung von R sind Vinyl, Propenyl oder Butenyl.

Bevorzugte gegebenenfalls substituierte C₆-C₂₀-Aryle in der Bedeutung von R sind Phenyl, Tolyl oder Naphthyl.

Bevorzugte gegebenenfalls substituierte C₃-C₂₀-Cycloalkyle in der Bedeutung von R sind Cyclopropyl, Cyclobutyl, Cyclopentyl oder Cyclohexyl.

Als bevorzugte Substituenten für die oben genannten Alkenyl-, Aryl und Cycloalkylreste kommen Methyl, Ethyl, n-Propyl, iso-Propoyl, Chlor, Brom, Fluor, Methoxy, Ethoxy, Dimethylamino, Methoxycarbonyl, Ethoxycarbonyl, Hydroxy und Acyl in Frage.

Als bevorzugte Substituenten für den oben genannten Alkylrest kommen Chlor, Brom, Fluor, Methoxy, Ethoxy, Dimethylamino, Methoxycarbonyl, Ethoxycarbonyl, Hydroxy, Acyl und Phenyl in Frage.

Bevorzugt sind Verbindungen der Formel (I), in denen R in der Bedeutung des C₁-C₂₀-Alkylrestes für gegebenenfalls substituiertes C₇-C₂₀-Alkylaryl, insbesondere Benzyl oder Phenylethyl oder C₁-C₆-Alkyl, wie Methyl, Ethyl, n-Propyl, iso-Propoyl oder n-Butyl und n für 1 steht. Besonders bevorzugt steht R für n-Butyl und n für 1.

Bevorzugt ist es, das erfindungsgemäße Verfahren in Wasser und/oder einem organischen Lösungsmittel durchzuführen. Im Falle von Mischungen beträgt das Verhältnis von Wasser zu organischem Lösungsmittel vorzugsweise 9:1 bis 1:9 insbesondere 3:1 bis 1:3

In Frage kommen verschiedene organische Lösungsmittel, wie z.B. aliphatische Kohlenwasserstoffe, aromatische Kohlenwasserstoffe, Alkohole, Ether, chlorierte Kohlenwassertoffe, Nitrile, Ester oder Ketone. Auch Mischungen von zwei oder mehr Lösungsmitteln können eingesetzt werden.

Desweiteren kann Wasser allein verwendet werden, gegebenenfalls in Kombination mit einem oder mehreren organischen Lösungsmitteln, insbesondere aliphatischen Alkoholen, vorzugsweise Methanol und/oder Ethanol.

Bei Verwendung von Wasser ggf. zusammen mit einem organischen Lösungsmittel können oberflächenaktive Substanzen mitverwendet werden, z.B. nichtionische, anionische, amphotere oder kationische Emulgatoren, wie z.B. Betaine, Polyoxyethylen-Fettsäureester, Polyoxyethylen-Fettalkoholether, Alkylarylpolyglycolether, Alkylsulfonate, Alkylsulfate, Arylsulfonate sowie Alkoholethoxylate, insbesondere Ethoxylate von C₁₀-C₁₈-Alkoholen. Weiterhin können Phasentransferkatalysatoren verwendet werden, wie z.B. Tetrabutylammoniumhydrogensulfat, Tetrabutylammoniumbromid, Tetrabutylammoniumchlorid, Tetraoctylammoniumbromid, Tetraoctylammoniumchlorid, Methyltridecylammoniumchlorid, Methyltrioctylammoniumchlorid (Aliquat 336) oder Methyltributylammoniumchlorid.

Als Iodquelle kommt elementares Iod (I₂) und/oder Metalliodide, wie beispielsweise Natriumiodid, Kaliumiodid, oder Mischungen in Frage. Diese Verbindungen können als Feststoff oder gelöst in einem geeigneten Lösungsmittel, insbesondere Alkohole wie Methanol oder Ethanol und/oder Wasser eingesetzt werden.

Im Falle von Iod (I₂) wird dieses vorzugsweise in einer Menge von 0,4 bis 0,75 mol-Äquivalenten, insbesondere 0,45 bis 0,6 mol-Äquivalenten, bezogen auf die eingesetzte Propargylverbindung der Formel (II), eingesetzt. Im Falle von Metalliodid wird dieses vorzugsweise in einer Menge von 0,8 bis 1,5 mol-Äquivalenten, insbesondere 0,9 bis 1,2 mol-Äquivalenten, bezogen auf die eingesetzte Propargylverbindung der Formel (II), eingesetzt.

Dabei kann die Gesamtmenge an Iod bzw. Metalliodid bereits bei Beginn der Reaktion vorliegen oder nur ein Teil, wobei der Rest kontinuierlich oder diskontinuierlich z.B. portionsweise im Laufe der Umsetzung zugegeben wird.

Das erfindungsgemäße Verfahren wird in Anwesenheit einer Base durchgeführt. Als Base eignet sich beispielsweise Alkalihydroxide, Phosphate, Alkoholate und Carbonate sowie Mischungen davon. Besonders geeignet sind aus der Reihe der Alkalihydroxide NaOH und KOH sowie aus der Reihe der Carbonate Na₂CO₃, K₂CO₃, MgCO₃. Ganz besonders bevorzugt werden wässrige Lösungen von NaOH und/oder KOH eingesetzt. Die Base wird vorzugsweise, bezogen auf die Propargylverbindung der Formel (II), in einem molaren Verhältnis von 1 : 1 bis 10 : 1, bevorzugt von 1 : 1 bis 5 : 1 eingesetzt.

Das eingesetzte Chlor wird vorzugsweise in einer Menge von 0,7 bis 5 mol-Äquivalenten, vorzugsweise 1 bis 2 mol-Äquivalenten, bezogen auf eingesetztes Iod (I₂) oder Metalliodid eingesetzt.

Das Chlor wird vorzugsweise während der Umsetzung in das Reaktionsmedium als Gas eingeleitet. Die Geschwindigkeit wird vorzugsweise so gewählt, dass das erfindungsgemäße Verfahren vorzugsweise bei -20 bis +30°C, insbesondere bei -10 bis +10°C, besonders bevorzugt bei 0 bis 10°C durchgeführt wird.

Zur Durchführung des erfindungsgemäßen Verfahrens geht man im allgemeinen so vor, dass man die Propargylverbindung der Formel (II) in einem Lösungsmittel vorlegt, vorzugsweise die Reaktionstemperatur einstellt, anschließend die Base und ggfs. weitere Additive zugibt und danach die Iodquelle vollständig oder nach und nach in geeigneter Geschwindigkeit, entweder als Feststoff oder gelöst in einem geeigneten Lösungsmittel, zudosiert. Anschließend wird vorzugsweise in geeigneter Geschwindigkeit das Chlor in das Reaktionsmedium eingeleitet, so dass die Reaktionstemperatur vorzugsweise die gewünschte Temperatur nicht übersteigt. Nach beendeter Reaktion kann je nach verwendetem Reaktionsmedium zur Aufarbeitung die Verbindung der Formel (I) z.B. mittels Extraktion mit einem mit dem Reaktionsmedium nicht mischbaren Lösungsmittel isoliert werden oder direkt aus dem Reaktionsmedium durch Filtration. Bei Verwendung von Wasser unter Mitverwendung eines organischen Lösungsmittels ist es auch möglich, das organische Lösungsmittel ganz oder teilweise durch Destillation zu entfernen und die ausgefallene Verbindung der Formel (I) anschließend durch Filtration zu isolieren.

Erforderlichenfalls kann die erhaltene Verbindung der Formel (I) noch umkristallisiert werden. Bevorzugt verwendet man dafür Mischungen aus aliphatischen Alkoholen, wie Methanol oder Ethanol und Wasser.

Das erfindungsgemäße Verfahren führt zu deutlich geringeren Abwasserfrachten gegenüber nur verdünnt einsetzbarem Oxidationsmittel wie Hypochlorit. Überraschend ist auch, dass das reaktive Chlor nicht mit der Dreifachbindung reagiert und keine störenden Nebenprodukte bildet. Es wurden keine Chloradditionsverbindungen an die Dreifachbindung gefunden.

Folgende Beispiele dienen der Veranschaulichung des erfindungsgemäßen Verfahrens ohne dieses jedoch einzuschränken.

### Beispiele

### Beispiel 1

Bei 5°C werden 4,5 g C₁₂-C₁₆-Alkoholethoxylat, 16,9 g Propargylbutylcarbamat (0,107 mol), 40,7 g NaI-Lösung (40%-ig, 0,108 mol) und 25,8 g NaOH-Lösung (50%-ig, 0,322 mol) in 330 g Wasser vorgelegt. Anschließend wird langsam Chlor (10,3 g, 0,145 mol) in die Reaktionsmischung eingeleitet, so dass die Temperatur unter 5°C bleibt. Nach Beendigung des Einleitens wird langsam auf 20°C erwärmt und 4 h bei dieser Temperatur gerührt. Anschließend wird der ausgefallene Feststoff durch Filtration isoliert, mit Wasser gewaschen und im Vakuum getrocknet. Man erhält 26,1 g 3-Iod-2-propinyl-N-butylcarbamat (Reinheit: 95,4% (HPLC), mehrfach iodierte Verbindungen: <0,1 % (HPLC), Ausbeute: 83%).

### Beispiel 2

Es werden 10,1 g Natriumhydroxid (0,244 mol) und 19,0 g Propargylbutylcarbamat (0,122 mol) bei 8°C in 48 g Methanol und 50 g Wasser vorgelegt. Bei dieser Temperatur werden portionsweise 15,5 g Iod (0,061 mol) zugegeben. Anschließend wird langsam Chlor (5,6 g, 0,079 mol) in die Reaktionsmischung eingeleitet, so dass die Temperatur weiterhin unter 8°C bleibt. Nach beendeter Dosierung wird 1 h bei dieser Temperatur gerührt und anschließend 105 g Wasser zugegeben. Der ausgefallene Feststoff wird durch Filtration isoliert, mit Wasser gewaschen und im Vakuum getrocknet. Man erhält 28,5 g 3-Iod-2-propinyl-N-butylcarbamat (Reinheit: 95,0% (HPLC), mehrfach iodierte Verbindungen: 0,3 % (HPLC), Ausbeute: 79%).

### Beispiel 3

Bei 5°C werden 19,0 g Propargylbutylcarbamat (0,122 mol), 12,2 g Natriumhydroxid (0,305 mol) und 18,5 g Natriumiodid (0,123 mol) in 50 g Wasser und 48 g Methanol vorgelegt. Anschließend wird langsam Chlor (11,7 g, 0,165 mmol) eingeleitet, so dass die Temperatur unter 5°C bleibt. Nach beendeter Dosierung wird 1 h bei dieser Temperatur gerührt, anschließend langsam auf Raumtemperatur erwärmt und 1 Stunde gerührt. Nach Zugabe von 100 g Wasser wird der ausgefallene Feststoff durch Filtration isoliert, mit Wasser gewaschen und im Vakuum getrocknet. Man erhält 29,1 g 3-Iod-2-propinyl-N-butylcarbamat (Reinheit: 94,7% (HPLC), mehrfach iodierte Verbindungen: <0,1 % (HPLC), Ausbeute: 80%).

## Patentansprüche

1. Verfahren zu Herstellung von Iodpropargylverbindungen der Formel (I), 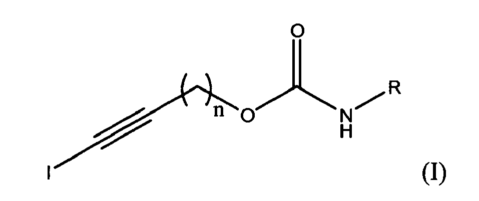 worin R für Wasserstoff, jeweils gegebenenfalls substituiertes C₁-C₂₀-Alkyl, C₂-C₂₀-Alkenyl, C₆-C₂₀-Aryl oder C₃-C₂₀-Cycloalkyl
und n für eine ganze Zahl von 1 bis 6 steht,
**dadurch gekennzeichnet, dass** man Propargylverbindungen der Formel (II) 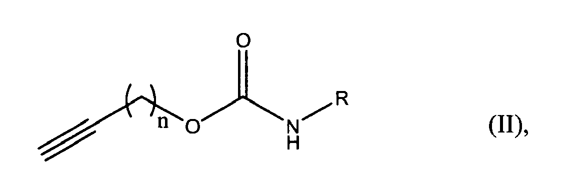 worin R und n die obige Bedeutung haben, mit Iod und/oder Metalliodiden in Gegenwart einer Base und unter Verwendung von Chlor umsetzt.

2. Verfahren gemäß Anspruch 1, **dadurch gekennzeichnet, dass** R für C₁-C₆-Alkyl steht und n für 1 steht.

3. Verfahren gemäß Anspruch 1, **dadurch gekennzeichnet, dass** R für n-Butyl und n für 1 steht.

4. Verfahren gemäß Anspruch 1, **dadurch gekennzeichnet, dass** die Umsetzung mit Iod erfolgt.

5. Verfahren gemäß Anspruch 1, **dadurch gekennzeichnet, dass** die Umsetzung mit Metalliodiden, insbesondere mit NaI und/oder KI erfolgt.

6. Verfahren gemäß Anspruch 1, **dadurch gekennzeichnet, dass** die Umsetzung in Wasser und/oder einem organischen Lösungsmittel erfolgt.

7. Verfahren gemäß Anspruch 1, **dadurch gekennzeichnet, dass** die Umsetzung in einem wässrigen Medium enthaltend wenigstens ein organisches Lösungsmittel erfolgt.

## Claims

1. Process for the preparation of iodopropargyl compounds of the formula (I), 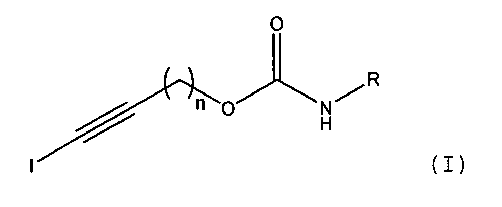 in which R is hydrogen, in each case optionally substituted C₁-C₂₀-alkyl, C₂-C₂₀-alkenyl, C₆-C₂₀-aryl or C₃-C₂₀-cycloalkyl
and n is an integer from 1 to 6,
**characterized in that** propargyl compounds of the formula (II) 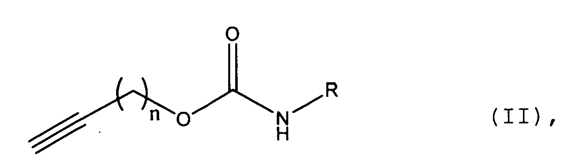 in which R and n have the above meaning, are reacted with iodine and/or metal iodides in the presence of a base and using chlorine.

2. Process according to Claim 1, **characterized in that** R is C₁-C₆-alkyl and n is 1.

3. Process according to Claim 1, **characterized in that** R is n-butyl and n is 1.

4. Process according to Claim 1, **characterized in that** the reaction takes place with iodine.

5. Process according to Claim 1, **characterized in that** the reaction takes place with metal iodides, in particular with NaI and/or KI.

6. Process according to Claim 1, **characterized in that** the reaction takes place in water and/or an organic solvent.

7. Process according to Claim 1, **characterized in that** the reaction takes place in an aqueous medium comprising at least one organic solvent.

## Revendications

1. Procédé pour la préparation de composés de type iodopropargyle de formule (I), 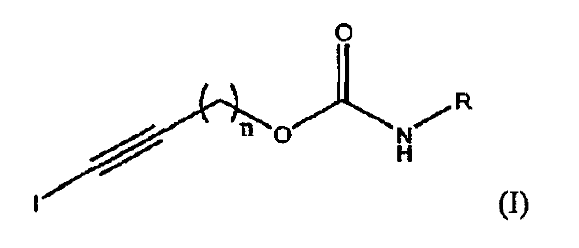 dans laquelle R représente un atome d'hydrogène, un groupe alkyle en C₁-C₂₀, alcényle en C₂-C₂₀, aryle en C₆-C₂₀ ou cycloalkyle en C₃-C₂₀, chacun éventuellement substitué
et n représente un nombre entier valant de 1 à 6,
**caractérisé en ce qu'**on fait réagir des composés de type propargyle de formule (II) 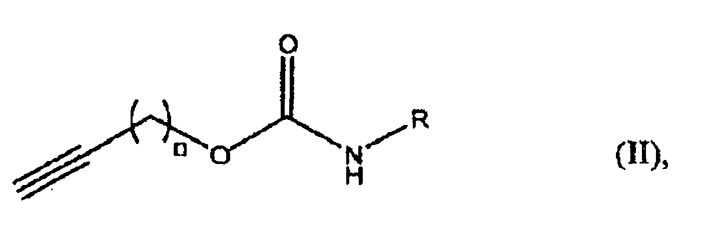 dans laquelle R et n ont la signification ci-dessus, avec de l'iode et/ou des iodures métalliques en présence d'une base et avec utilisation de chlore.

2. Procédé selon la revendication 1, **caractérisé en ce que** R représente un groupe alkyle en C₁-C₆ et n représente 1.

3. Procédé selon la revendication 1, **caractérisé en ce que** R représente le groupe n-butyle et n représente 1.

4. Procédé selon la revendication 1, **caractérisé en ce que** la réaction s'effectue avec de l'iode.

5. Procédé selon la revendication 1, **caractérisé en ce que** la réaction s'effectue avec des iodures métalliques, en particulier avec NaI et/ou KI.

6. Procédé selon la revendication 1, **caractérisé en ce que** la réaction s'effectue dans de l'eau et/ou dans de l'eau et/ou un solvant organique.

7. Procédé selon la revendication 1, **caractérisé en ce que** la réaction s'effectue dans un milieu aqueux contenant au moins un solvant organique.
